# EUROPÄISCHE PATENTSCHRIFT

(11) **EP 2 663 258 B1**
(45) Veröffentlichungstag und Bekanntmachung des Hinweises auf die Patenterteilung: **21.11.2018**
(21) Anmeldenummer: 11826112.2
(22) Anmeldetag: 22.12.2011
(51) Int. Cl.: A61F 2/24

(54) **KLAPPENPROTHESE ZUM ERSATZ EINER ARTRIOVENTRIKULARKLAPPE DES HERZENS**
PROSTHETIC VALVE FOR REPLACING AN ATRIOVENTRICULAR HEART VALVE
PROTHÈSE VALVULAIRE DESTINÉE À REMPLACER UNE VALVULE AURICULO-VENTRICULAIRE DU COEUR

(30) Priorität: 11.01.2011 WO PCT/EP2011/000082
(43) Veröffentlichungstag der Anmeldung: 20.11.2013
(73) Patentinhaber: Figulla, Hans Reiner, 07749 Jena (DE); Lauten, Alexander, 14532 Kleinmachnow (DE)
(72) Erfinder: Figulla, Hans Reiner, 07749 Jena (DE); Lauten, Alexander, 14532 Kleinmachnow (DE)
(74) Vertreter: Trinks, Ole
(86) Internationale Anmeldenummer: PCT/EP2011/006573
(87) Internationale Veröffentlichungsnummer: WO 2012/095159

(56) Entgegenhaltungen:
- WO-A1-2011/057087
- WO-A2-2010/121076
- US-A1- 2006 058 872
- US-A1- 2009 276 040

## Beschreibung

Die Erfindung betrifft eine Klappenprothese zum Ersatz einer Artrioventrikularklappe des Herzens, also der Mitralklappe oder der Trikuspidalklappe.

Bei Patienten mit einer Funktionsbeeinträchtigung einer Herzklappe ist ein offener chirurgischer Eingriff am Herzen zum Einsatz einer Klappenprothese (Ersatzklappe) aufgrund des Allgemeinzustandes des Patienten häufig mit erhöhten Risiken verbunden, sodass Herzklappenprothesen zunehmend minimal invasiv über einen Katheter implantiert werden.

Der Stand der Technik kennt hierfür den Einsatz von Stents, in denen Ersatz-Herzklappensegel befestigt sind und die zur Einführung über einen Katheter stark komprimierbar sind und so durch den Katheter an den Ort der zu ersetzenden Herzklappe vorschiebbar und dort freisetzbar sind. Der zum Beispiel ballonexpandierbare oder selbstexpandierbare Stent entwickelt im freigesetzten Zustand eine radiale Spreizkraft, die eine Verankerung der Ersatz-Klappenprothese bewirkt oder zumindest fördert. Für eine solche Verankerung der Klappenprothese ist eine Ersatz-Aortenklappe besonders geeignet, die mit radialer Spreizkraft am Ort der dysfunktionalen Aortenklappe verankert werden kann. Zum Stand der Technik hierzu vgl. z.B. EP 1 994 913 A2; EP 1 469 797 B1; EP 1 259 195 B1; WO 2007/051620 A1;
WO 2007/048529 A1; EP 1 980 220 A1; WO 01/64137 A1; EP 1 255 510 B3; und US 5,411,552. Klappenprothesen sind auch bekannt aus WO 2011/057087 A1; WO 2010/121076 A2; US 2006/058872 A1; US 2009/276040 A1.

Die Mitralklappe des Herzens, also die Klappe zwischen dem linken Vorhof (Atrium) und der linken Kammer (Ventrikel) ist allerdings wenig geeignet für eine Ersatzprothese, die wesentlich durch radiale Spreizkraft eines Stents kraftschlüssig (durch Reibung) vor Ort verankert ist, da der Annulus der Mitralklappe durch seine Flexibilität nur ein unzureichendes Widerlager für die Prothesenverankerung bietet.

Der Erfindung liegt die Aufgabe zu Grunde, eine Klappenprothese zum Ersatz einer Artrioventricularklappe bereitzustellen, die mittels eines Katheters implantierbar ist und eine stabile sowie orthotrope Positionierung und Verankerung ermöglicht.

Die Erfindung betrifft eine Klappenprothese gemäß dem unabhängigen Patentanspruch 1, wobei vorteilhafte Weiterbildungen der erfindungsgemäßen Klappenprothese in den abhängigen Ansprüchen angegeben sind.

Eine erfindungsgemäße Klappenprothese zum Ersatz einer Artrioventrikularklappe des Herzens ist unter anderem versehen mit
- einem Ringkörper, an dem Herzklappensegel befestigt sind und der adaptiert ist, um in einen Klappenannulus des Herzens eingesetzt zu werden, und mit
- mehreren Ankerelementen, die mit dem Ringkörper ventrikelseitig verbunden sind,
wobei
- die Ankerelemente sich radial außenseitig des Ringkörpers und im Wesentlichen parallel zu dessen Außenwand erstrecken.

Die genannten Ankerelemente (die auch als Fühler oder Arme bezeichnet werden können) erstrecken sich entlang der Außenseite des genannten Ringkörpers bis zum Klappenannulus des Herzens, sodass mittels dieser Ankerelemente die Klappenprothese insgesamt abgestützt wird, insbesondere gegen axial wirkenden Kräfte im Schließzustand der Ersatz-Artrioventrikularklappe.

Dabei ist die axiale Länge des genannten Ringkörpers in Bezug auf die vorstehend beschriebene Länge der Ankerelemente bevorzugt so bemessen, dass sich die Ankerelemente über zumindest die Hälfte der axialen Länge des Ringkörpers erstrecken.

Gemäß der Erfindung sind die genannten Ankerelemente direkt am ventrikelseitigen Ende des Ringkörpers in axialer Richtung abgebogen. Somit stehen die Ankerelemente nicht wesentlich in Ventrikelrichtung vom Ringkörper vor, sondern erstrecken sich im Wesentlichen radial außerhalb des Ringkörpers mit geringem radialen Abstand vom Ringkörper. Dieser geringe radiale Abstand der Ankerelemente von der Außenwand des Ringkörpers ist so bemessen, dass die Ankerelemente zwischen sich und der Außenwand des Ringkörpers insbesondere Gewebe von Klappensegeln und Sehnenfäden einklemmen. Durch diese Klemmwirkung der Ankerelemente wird die stabile Positionierung der Ersatz-Artrioventrikularklappe weiter gefördert. Damit die Ankerelemente keine unerwünschte Reibwirkung mit Herzgewebe, insbesondere im Bereich des Annulus, haben, sind die Ankerelemente an ihren freistehenden axialen Enden abgerundet, zum Beispiel indem sie in einen Ring übergehen.

Gemäß der Erfindung hat die Ersatz-Klappenprothese auch atriumseitig Ankerelemente, die analog den oben beschriebenen Ankerelementen auf der anderen Seite (d.h. atriumseitig) mit dem Ringkörper verbunden sind und sich außenseitig des Ringkörpers und im Wesentlichen parallel zu dessen Außenwand erstrecken. Diese Erstreckung reicht bevorzugt auch bis zum Annulus des Herzens, sodass die Klappenprothese durch die ventrikelseitigen Ankerelemente und die atriumseitigen Ankerelemente quasi am Annulus allseitig fixiert und abgestützt wird.

Eine bevorzugte Ausgestaltung der Erfindung sieht vor, dass der genannte Ringkörper etwa kegelstumpfförmig ist, wobei bevorzugt der atriumseitige Durchmesser des Kegelstumpfes größer ist als der ventrikelseitige Durchmesser des Kegelstumpfes. Eine andere Ausgestaltung sieht vor, dass der Ringkörper kegelstumpfförmig und/oder im Durchmesser elliptisch ausgeführt wird (entsprechend der elliptischen Zirkumferenz der AV-Klappen).

Der Ringkörper kann zum Beispiel aus einem Drahtgeflecht geformt sein, insbesondere aus zick-zack-förmigen Drähten, wie es grundsätzlich in der Stent-Technologie als solches bekannt ist.

Weiterhin sind die genannten Ankerelemente bevorzugt integral mit dem Drahtgeflecht des Ringkörpers ausgeformt. Die Zahl der Ankerelemente auf zum Beispiel der Ventrikelseite des Ringkörpers sollte mindestens drei, bevorzugt aber vier oder mehr betragen. Entsprechendes gilt gegebenenfalls für die Zahl der Ankerelemente auf der Atriumseite des Ringkörpers.

Dabei sind die Ankerelemente bevorzugt unter regelmäßigen Abständen angeordnet, bei Einsatz von vier Ankerelementen also jeweils unter einem Winkel von 90° beabstandet.

Als Material für den Ringkörper und die Ankerelemente kommt ein geeignetes Metall oder eine Metalllegierung in Betracht, zum Beispiel wie sie dem Fachmann aus der Stent-Technologie bekannt sind, oder auch ein Metallgeflecht z. B. aus einem Gedächtnismetall.

In der Systole ist das Ventrikel gegen das Atrium abzudichten. Diese Abdichtung leistet die Klappenprothese. Die dabei auf die Herzklappensegel der Prothese wirkende Kraft entspricht dem vollen Blutdruck. Gegen diese Kraft muss die Klappenprothese stabil und dauerhaft orthotrop verankert werden. Die Erfindung nutzt die Erkenntnis, dass diese Kraft im Wesentlichen in Richtung der zentralen Achse des Ringkörpers (entsprechend in Richtung der zentralen Achse des Annulus) wirkt, sodass die Verankerung entsprechende Zugkräfte in dieser Richtung auffangen muss. Deshalb kann die erfindungsgemäße Klappenprothese darauf verzichten, dass wesentliche Verankerungskräfte durch Reibschluss zwischen der Klappenprothese und dem Annulus erzeugt werden. Vielmehr wird die Klappenprothese während der Systole, in der sie den Schließzustand einnimmt, über in axialer Richtung wirkende Stützkräfte gehalten, die über die Ankerelemente in das umliegende Gewebe, also insbesondere in die Ventrikelseite des Annulus eingeleitet werden. Darüber hinaus kann durch eine Klemmwirkung zwischen den Ankerelementen und Mitralklappensegeln bzw. Sehnenfäden die Abstützung und Stabilisierung der Klappenprothese gefördert werden.

Die Spreizkraft des Ringkörpers ist hinsichtlich seiner Abmessung und seiner elastischen Eigenschaften so gewählt, dass bei Einsatz im Klappenannulus des Herzens keine den Klappenannulus wesentlich aufweitenden radialen Spreizkräfte wirken.

Herzklappensegel, die mit der erfindungsgemäßen Klappenprothese eingesetzt werden, können gemäß dem Stand der Technik ausgewählt und am oder im Ringkörper befestigt werden, z.B. US 5,411,552 oder EP 1 255 510 B3. Zum Beispiel kann eine von einem Schwein gewonnene biologische Herzklappe oder eine aus Perikard geformte Klappe in den Ringkörper eingenäht werden.

Die Erfindung stellt eine Herzklappenprothese bereit, deren ventrikelseitige Verankerung eine stabile und orthotrope Positionierung ermöglicht. Die Verankerung verhindert eine Verschiebung der Klappenprothese in axialer Richtung. Wenn hier von axialer oder radialer Richtung sowie Umfangsrichtung gesprochen wird, bezieht sich dies auf den genannten Ringkörper der Klappenprothese beziehungsweise, im implantierten Zustand, auf den Annulus der ersetzten Mitral- bzw. Trikuspidalklappe.

Die erfindungsgemäße Artrioventricularersatzklappe ist ballonexpandierbar oder selbstexpandierbar.

Implantierbar ist die Ersatzklappe mittels eines Katheters transepital, transapikal über die Herzspitze, oder retrograd durch die Aorta.

Die Positionierung der Klappenprothese mittels eines Katheters erfolgt bevorzugt derart, dass die beschriebenen Ankerelemente als erstes am Katheterende freigesetzt werden und sodann mit ihren Ankerelementen hinter den Mitraisegeln und/oder zwischen den Sehnenfäden verankert und abgestützt werden. Sodann wird die Klappenprothese vollständig auch mit dem Ringkörper freigesetzt, derart, dass der Ringkörper mit seiner Mitte etwa auf dem Annulus der ersetzten Klappe zu liegen kommt.

Die erfindungsgemäßen Ankerelemente sind so gestaltet, dass zwischen ihnen im Ventrikel der Zugang zur Aorta und der Aortenklappe für eine ungehinderte Blutströmung frei bleibt.

Gemäß der Erfindung ist ferner vorgesehen, dass die axiale Länge der Ankerelemente einstellbar ist, z. B. über eine arretierbare Teleskopanordnung, eine Schraubverbindung oder dergleichen, sodass der Chirurg vor Ort eine Anpassung an die anatomischen Gegebenheiten vornehmen kann.

Nachfolgend werden Ausführungsbeispiele der Erfindung anhand der Zeichnungen näher erläutert. Es zeigt:
- Figur 1: schematische eine Klappenprothese zum Ersatz einer Artrioventrikularklappe des Herzens;
- Figur 2: den Einsatz der Klappenprothese in einem Herzen zwischen Artrium und Ventrikel;
- Figur 3: eine Seitenwand des Ringkörpers der Klappenprothese mit Ankerelementen und die Verankerung im Bereich des Annulus des Herzens;
- Figur 4: schematisch ein anderes Ausführungsbeispiel einer Klappenprothese zum Ersatz einer Artrioventrikularklappe des Herzens;
- Figur 5: eine Klappenprothese gemäß Figur 4 in implantiertem Zustand im Herzen; und
- Figur 6: eine Klappenprothese gemäß den Figuren 4 und 5 in vergrößertem Maßstab.

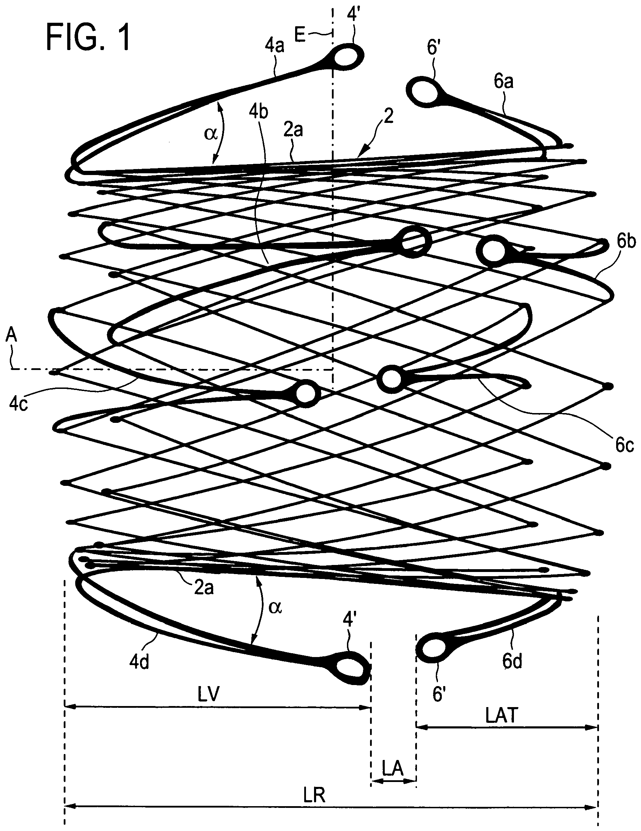

Die in den Figuren 1, 2 und 3 dargestellte Klappenprothese zum Ersatz einer Artrioventrikularklappe des Herzens weist einen Ringkörper 2 auf, in dem nicht näher gezeigte Herzklappensegel befestigt sind. Für die Herzklappensegel können als solches bekannte Systeme des Standes der Technik eingesetzt werden, zum Beispiel Segel aus Perikardium, oder zum Beispiel vom Herzen eines Schweins gewonnene Herzklappen, die in dem Ringkörper 2 vernäht sind. Entsprechender Stand der Technik ist oben exemplarisch angegeben. Der Ringkörper 2 ist aus einem Drahtgeflecht gebildet und die elastischen Eigenschaften des Drahtes sowie die Abmessungen des Ringkörpers 2 sind so gewählt, dass der Ringkörper im Einsatz der Klappenprothese als Mitralklappe oder Trikuspidalklappe - anders als üblicherweise ein Stent - keine den natürlichen Klappenannulus wesentlich aufweitende radiale Spreizkraft erzeugt.

Für das Drahtgeflecht des Ringkörpers 2 kann ein Metall mit Formgedächtnis eingesetzt werden, zum Beispiel Nitinol.

Wie die Figuren zeigen, ist der Ringkörper 2 rotationssymmetrisch um seine Achse A. Beim in den Figuren 1 bis 3 gezeigten Ausführungsbeispiel ist der Ringkörper 2 kegelstumpfförmig ausgebildet mit etwas größerem Durchmesser auf der Artriumseite (in Figur 1 rechts) als auf der Ventrikelseite (Figur 1, links). In Abwandlung des vorstehend beschriebenen Ausführungsbeispiels mit rotationssymmetrischem Ringkörper kann der Ringkörper auch eine elliptische Zirkumferenz (Umfang) aufweisen.

Auf der Ventrikelseite (Figur 1, links) geht das Drahtgeflecht des Ringkörpers 2 integral in vier Ankerelemente 4a, 4b, 4c, 4d über. Die Ankerelemente erstrecken sich, ausgehend vom ventrikelseitigen Ende des Ringkörpers 2, direkt radial außerhalb der Außenwand 2a des Ringkörpers 2 in Richtung auf das Zentrum des Ringkörpers 2. Dabei schließen die armförmigen Ankerelemente 4a, 4b, 4c, 4d mit der Außenwand 2a des Ringkörpers 2 einen Winkel α ein. Jedes Ankerelement ist mit zwei in Umfangsrichtung voneinander beabstandeten Streben am Ringkörper befestigt, um jedes Ankerelement stabil in Bezug auf den Ringkörper zu positionieren.

An ihren freistehenden, über die radiale Mittelebene E des Ringkörpers 2 in Richtung des Atriums vorstehenden Enden sind die Ankerelemente 4a, 4b, 4c, 4d abgerundet, um Reibwirkung mit Gewebe zu vermeiden. Beim dargestellten Ausführungsbeispiel wird die Abrundung dadurch erreicht, dass die Ankerelemente in Ringe 4' übergehen.

Weiterhin zeigt das Ausführungsbeispiel gemäß den Figuren 1 bis 3, dass auch auf der Atriumseite des Ringkörpers 2 Ankerelemente 6a, 6b, 6c, 6d analog den oben beschriebenen ventrikelseitigen Ankerelementen mit dem Ringkörper 2 verbunden sind. Auch diese Ankerelemente 6a, 6b, 6c, 6d gehen an ihren in Richtung auf die radiale Mittelebene des Ringkörpers weisenden freien Enden in Abrundungen z.B. in Form von Ringen 6' über.

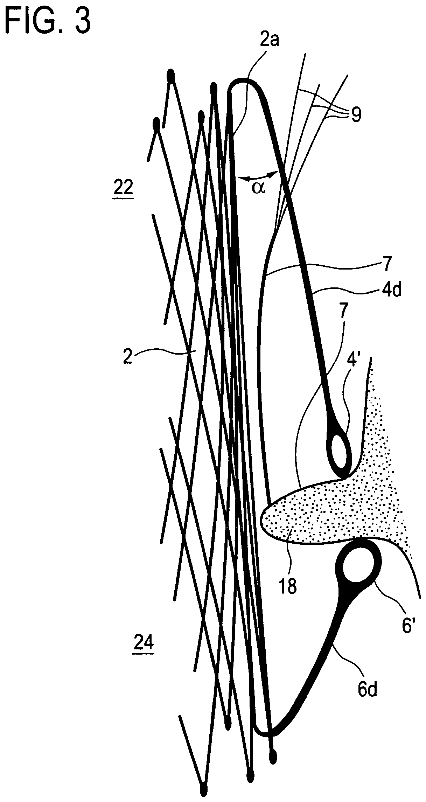

Figur 1 zeigt auch die axialen Abmessungen der Komponenten. Die axiale Länge LV der ventrikelseitigen Ankerelemente 4a, 4b, 4c ist so bemessen, dass sie über die radiale Mittelebene E (Figur 1) hinausragen und im Einbauzustand der Klappenprothese mit ihren abgerundeten Enden am Annulus des Herzens anliegen (vgl. Figur 3).

Die axiale Länge LAT der atriumseitigen Ankerelemente 6a, 6b, 6c, 6d ist so bemessen, dass sie mit ihren abgerundeten freien Enden auf der anderen Seite des Annulus, also atriumseitig, anliegen.

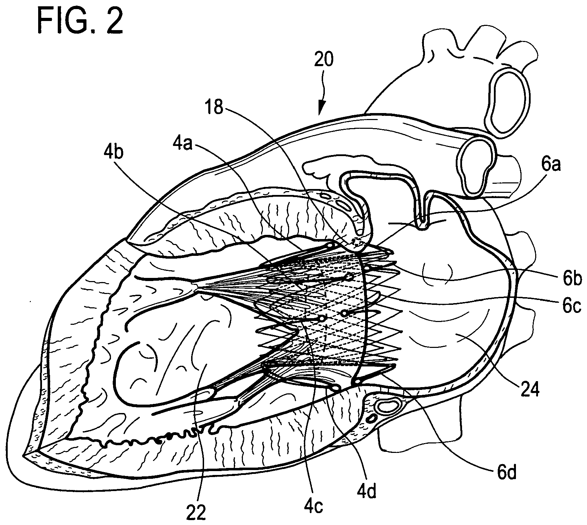

Somit ist der axiale Abstand zwischen den Enden 4' der ventrikelseitigen Ankerelemente und den Enden 6' der artriumseitigen Ankerelemente so bemessen, dass er etwa der axialen Stärke LA des Annulus entspricht. Die Gesamtlänge LR des Ringkörpers ergibt sich aus Figur 2. Sie ist so bemessen, dass ventrikelseitig das Ende des Ringkörpers in den Bereich der Sehnenfäden der Herzklappe reicht, sodass die Ankerelemente 4a, 4b, 4c, 4d durch die Zwischenräume zwischen den Sehnenfäden gesteckt und so hinter das flächige Gewebe der Papillarmuskeln bis zum Annulus geschoben werden können. Aus diesen anatomischen Gegebenheiten, wie sie in Figur 2 dargestellt sind, folgt die axiale Länge LR der Klappenprothese.

Figur 3 zeigt die Außenwand 2a des Ringkörpers 2 auf einer Seite mit dem Annulus 18 der Herzklappe, Mitralsegel 7 und Sehnenfäden 9.

Wie vorstehend erläutert, werden die Ankerelemente 4d etc. zwischen den Sehnenfäden 9 hindurch hinter die Segel positioniert und kommen an ihren freien Enden 4' mit der Ventrikelseite des Annulus 18 in abstützende Anlage.

Der Winkel α zwischen den armartigen Ankerelementen und der Außenwand 2a des Ringkörpers 2 ist so bemessen, dass die Ankerelemente 4a, 4b, 4c, 4d das Papillarmuskelgewebe 7 zwischen sich und der Außenwand 2a des Ringkörpers einklemmen, sodass hierdurch die stabile Positionierung der Klappenprothese gefördert wird.

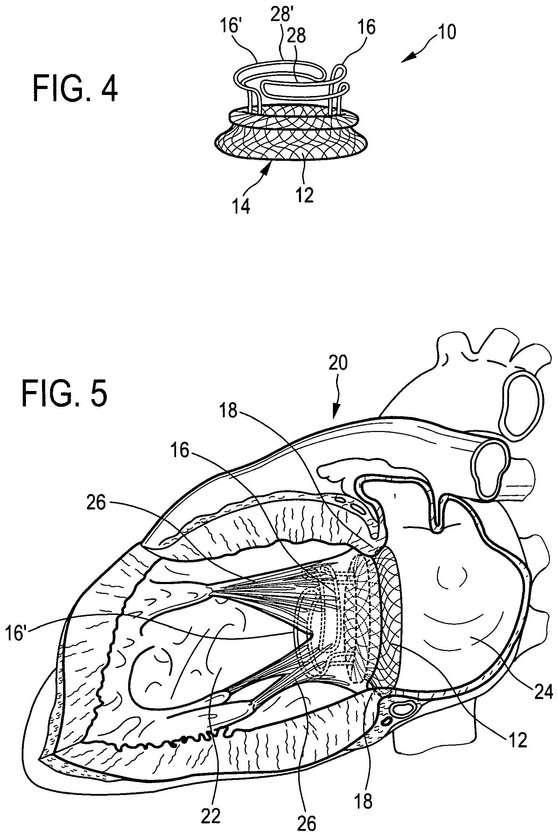

Die Figuren 4 bis 6 zeigen ein anderes, nicht erfindungsgemäßes Ausführungsbeispiel einer Klappenprothese.

Die in Figur 4 gezeigte Klappenprothese 10 zum Ersatz einer Atrioventricularklappe des Herzens weist einen Ringkörper 12 auf, in dem nicht näher gezeigte Herzklappensegel befestigt sind. Für die Herzklappensegel 14 können als solches bekannte Systeme des Standes der Technik eingesetzt werden, z. B. Segel aus Pericardium, oder z. B. vom Herz eines Schweins gewonnene Herzklappen, die in dem Ringkörper 12 vernäht werden. Der Ringkörper 12 ist aus einem Drahtgeflecht gebildet und die elastischen Eigenschaften des Drahtes und die Abmessungen des Ringkörpers 12 sind so gewählt, dass der Ringkörper im Einsatz der Klappenprothese als Mitralklappe oder Trikuspidalklappe - anders als üblicherweise ein Stent - keine den natürlichen Klappenannulus wesentlich aufweitende radiale Spreizkraft erzeugt.

Für das Drahtgeflecht des Ringkörpers 12 kann ein Metall mit Formgedächtnis eingesetzt werden, z. B. Nitinol.

Wie die Figuren 4, 5 und 63 zeigen, ist der Ringkörper rotationssymmetrisch um seine Achse A. Der atriumseitige Durchmesser D1 des Ringkörpers 12 ist größer als der ventrikelseitige Durchmesser D2 und dieser ist wiederum größer als ein mittiger Durchmesser D3 des Ringkörpers 12.

Wie die Figuren zeigen, stehen vom Ringkörper 12 ventrikelseitig Ankerelemente 16, 16' in axialer Richtung vor. Wie Figur 5 zeigt, ragen diese Ankerelemente 16, 16' in den Ventrikel 22. Der Ringkörper 12 wird mit seinem mittleren Abschnitt, also dem Abschnitt mit reduziertem Durchmesser D3, im Annulus 18 der Mitralklappe positioniert, Figur 52 zeigt also den Einsatz der Klappenprothese zwischen linkem Atrium und linkem Ventrikel. Der Bereich des Ringkörpers 12 mit vergrößertem Durchmesser D1 liegt satt am atriumseitigen Abschnitt des Annulus 18 an und fixiert die Klappenprothese in ihrem Offenzustand bei Blutströmung aus dem Atrium 24 in den Ventrikel 22.

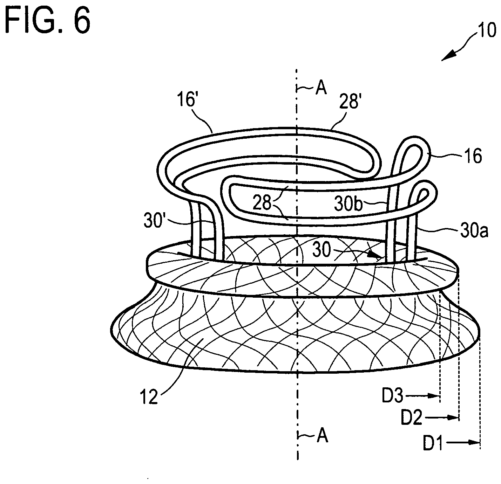

Die Ankerelemente 16, 16' weisen beim Ausführungsbeispiel jeweils zwei Komponenten auf, nämlich zum einen Arme 30a, 30b, die sich im Wesentlichen parallel zur Achse A des Ringkörpers 12 erstrecken, und Komponenten 28, 28', die sich hakenartig um die Achse A des Ringkörpers 12 gekrümmt in Umfangsrichtung des Ringkörpers 12 erstrecken, wie Figur 6 im Einzelnen zeigt.

Beim dargestellten Ausführungsbeispiel hat ein Arm 30 zur drehfesten Verbindung von Verankerungsteil 16 mit Ringkörper 12 zwei Armkomponenten 30a, 30b, die sich jeweils im Wesentlichen parallel zur Achse A des Ringkörpers erstrecken und an ihren ventrikelseitigen Enden über schlaufenartige Biegungen in die sich in Umfangsrichtung erstreckenden Komponenten 28, 28' der Ankerelemente übergehen. Die Ankerelemente 16, 16' sind aus einem geeignetem Metalldraht geformt.

Die Ankerelemente 16 sind insbesondere, je nach der gegebenen anatomischen Situation, so bemessen, dass der Abstand vom Annulus (18) zum ventrikelseitigen Ende des Verankerungsteils im Bereich von 5 bis 40 mm liegt, bevorzugt im Bereich von 5 bis 20 mm oder im Bereich von 10 bis 25 mm.

Ähnlich wie eine Stent-Klappenprothese ist die beschriebene Klappenprothese stark komprimierbar und über einen Katheter minimal-invasiv im Herzen positionierbar, nämlich insbesondere transepital, transapikal über die Herzspitze, oder auch retrograd über die Aorta. Hierfür können die als solche dem Fachmann bekannten Katheter-Techniken eingesetzt werden.

Das Verfahren zum Einsetzen der beschriebenen Klappenprothese sieht vor, dass die Ankerelemente 16, 16' als erstes am Einsatzort aus dem Katheter freigesetzt werden. Beim in den Figuren 4 bis 6 dargestellten Ausführungsbeispiel erfolgt dann die Verankerung der Ankerelemente 16' durch Drehung des Ringkörpers 12 mit den daran drehfest befestigten Ankerelementen 16, 16', welche sich dann mit ihren Komponenten 28, 28' z. B. in den Sehnenfäden oder auch in der nativen Seminularklappe verhaken. Die Drehung kann entweder durch Drehung des Katheters selbst erfolgen oder auch durch einen Pusher im Katheter, der einen Dreheingriff mit dem Ringkörper 12 erlaubt. Nach dieser Verhakung und Verankerung der Ankerelemente im Gewebe, z. B. der Sehnenfäden oder der nativen Seminularklappe, erfolgt dann die vollständige Freisetzung der Klappenprothese aus dem Katheter, wobei der diaboloförmige Ringkörper mit seinem mittleren Bereich reduzierten Durchmessers gemäß Figur 2 im Annulus 18 fixiert wird.

In Abwandlung des vorstehend beschriebenen Ausführungsbeispieles können die Verankerungskomponenten 28, 28' dahingehend abgewandelt werden, dass sie sich nicht in Umfangsrichtung erstrecken, sondern radial nach Außen, sodass sie durch Aufspreizung nach dem Freisetzen in Verankerungseingriff mit den Sehnenfäden bringbar sind oder auch in Verankerungseingriff mit nativen Segeln, je nach den anatomischen Gegebenheiten.

## Patentansprüche

1. Klappenprothese zum Ersatz einer Artrioventrikularklappe des Herzens mit
- einem Ringkörper (2), an dem Herzklappensegel befestigt sind und der adaptiert ist, um in einen Klappenannulus (18) des Herzens (20) eingesetzt zu werden, und mit
- mehreren Ankerelementen (4a, 4b, 4c, 4d), die mit dem Ringkörper (2) ventrikelseitig verbunden und direkt am ventrikelseitigen Ende des Ringkörpers (2) in axialer Richtung abgebogen sind, sodass die Ankerelemente (4a, 4b, 4c, 4d) nicht wesentlich in Ventrikelrichtung vom Ringkörper (2) vorstehen, sondern sich im Wesentlichen radial außerhalb des Ringkörpers (2) mit geringem radialen Abstand vom Ringkörper (2) erstrecken, wobei dieser geringe Abstand der Ankerelemente (4a, 4b, 4c, 4d) von der Außenwand des Ringkörpers (2) so bemessen ist, dass die Ankerelemente (4a, 4b, 4c, 4d) ausgebildet sind, im implantierten Zustand der Klappenprothese zwischen sich und der Außenwand des Ringkörpers (2) insbesondere Gewebe von Klappensegeln und Sehnenfäden einzuklemmen, und mit
- atriumseitigen Ankerelementen (6a, 6b, 6c, 6d), welche sich außerhalb des Ringkörpers (2) und im Wesentlichen parallel zu dessen Außenwand (2a) erstrecken und mit dem Ringkörper (2) analog den oben beschriebenen ventrikelseitigen Ankerelementen (4a, 4b, 4c, 4d) verbunden sind, **gekennzeichnet dadurch, dass** die axiale Länge (LV, LAT) der Ankerelemente (4a, 4b, 4c, 4d, 6a, 6b, 6c, 6d) einstellbar ist.

2. Klappenprothese nach Anspruch 1,
wobei die Ankerelemente (4a, 4b, 4c, 4d) sich ventrikelseitig entlang zumindest der Hälfte der axialen Länge (LR) des Ringkörpers (2) erstrecken.

3. Klappenprothese nach Anspruch 1 oder 2,
wobei die Ankerelemente (4a, 4b, 4c, 4d) an ihren Enden abgerundet sind, insbesondere in Ringform oder Teilringform.

4. Klappenprothese nach einem der vorhergehenden Ansprüche,
wobei die atriumseitig mit dem Ringkörper verbundenen Ankerelemente (6a, 6b, 6c, 6d) sich entlang weniger als der Hälfte der axialen Länge (LR) des Ringkörpers (2) erstrecken.

5. Klappenprothese nach einem der vorhergehenden Ansprüche,
wobei die atriumseitig mit dem Ringkörper (2) verbundenen Ankerelemente (6a, 6b, 6c, 6d) an ihren Enden abgerundet sind, insbesondere in Ringform oder Teilringform.

6. Klappenprothese nach einem der vorhergehenden Ansprüche,
wobei der Ringkörper (2) kegelstumpfförmig ist, insbesondere mit atriumseitig größerem Durchmesser als ventrikelseitig.

7. Klappenprothese nach einem der vorhergehenden Ansprüche,
wobei der Ringkörper (2) aus einem Drahtgeflecht besteht, insbesondere aus zick-zack-förmigen Drähten.

8. Klappenprothese nach einem der vorhergehenden Ansprüche,
wobei die Ankerelemente (4a, 4b, 4c, 4d; 6a, 6b, 6c, 6d) jeweils über zwei auf dem Umfang des Ringkörpers beabstandete Streben mit dem Ringkörper verbunden sind.

## Claims

1. A prosthetic valve for replacing an atrioventricular heart valve comprising:
- an annular body (2) to which heart valve leaflets are attached and which is adapted to be inserted into a valve annulus (18) of the heart (20), and comprising
- a plurality of anchoring elements (4a, 4b, 4c, 4d) connected to the annular body (2) at the ventricular side and bent in the axial direction directly at the ventricular end of the annular body (2) such that the anchoring elements (4a, 4b, 4c, 4d) do not substantially protrude from the annular body (2) in the direction of the ventricle but rather extend substantially radially from the annular body (2) at a small radial distance outside of said annular body (2), wherein said small distance of the anchoring elements (4a, 4b, 4c, 4d) from the outer wall of the annular body (2) is dimensioned such that in the implanted state of the prosthetic valve, the anchoring elements (4a, 4b, 4c, 4d) are designed to clamp in particular heart valve leaflet tissue and chordae tendineae between themselves and the outer wall of the annular body (2), and comprising
- atrial-side anchoring elements (6a, 6b, 6c, 6d) which extend outside of the annular body (2) and essentially parallel to its outer wall (2a) and are connected to the annular body (2) analogously to the above-described ventricle-side anchoring elements (4a, 4b, 4c, 4d),
**characterized in that**
the axial length (LV, LAT) of the anchoring elements (4a, 4b, 4c, 4d, 6a, 6b, 6c, 6d) is adjustable.

2. The prosthetic valve according to claim 1,
wherein the anchoring elements (4a, 4b, 4c, 4d) extend at the ventricular side over at least half of the axial length (LR) of the annular body (2).

3. The prosthetic valve according to claim 1 or 2,
wherein the anchoring elements (4a, 4b, 4c, 4d) are rounded at their ends, in particular in a ring shape or partial ring shape.

4. The prosthetic valve according to one of the preceding claims,
wherein the anchoring elements (6a, 6b, 6c, 6d) connected to the annular body (2) at the atrial side extend along less than half of the axial length (LR) of the annular body (2).

5. The prosthetic valve according to one of the preceding claims,
wherein the anchoring elements (6a, 6b, 6c, 6d) connected to the annular body (2) at the atrial side are rounded at their ends, in particular in a ring shape or partial ring shape.

6. The prosthetic valve according to one of the preceding claims,
wherein the annular body (2) is frustoconical, in particular with the atrial side having a larger diameter than the ventricular side.

7. The prosthetic valve according to one of the preceding claims,
wherein the annular body (2) consists of a wire mesh, in particular zig-zag-shaped wires.

8. The prosthetic valve according to one of the preceding claims,
wherein the anchoring elements (4a, 4b, 4c, 4d; 6a, 6b, 6c, 6d) are connected to the annular body via two struts respectively extending at a distance along the circumference of the annular body.

## Revendications

1. Prothèse valvulaire destinée à remplacer une valvule auriculo-ventriculaire du coeur, comportant
- un corps annulaire (2) auquel sont fixés des feuillets de valvule cardiaque et qui est adapté à être mis en place dans un anneau valvulaire (18) du coeur (20), et comportant
- plusieurs éléments d'ancrage (4a, 4b, 4c, 4d) qui sont reliés du côté ventriculaire au corps annulaire (2) et coudés en direction axiale directement à l'extrémité côté ventriculaire du corps annulaire (2), de sorte que les éléments d'ancrage (4a, 4b, 4c, 4d) ne font pas sensiblement saillie du corps annulaire (2) en direction ventriculaire, mais qu'ils s'étendent sensiblement radialement à l'extérieur du corps annulaire (2) à faible distance radiale du corps annulaire (2), et cette faible distance des éléments d'ancrage (4a, 4b, 4c, 4d) depuis la paroi extérieure du corps annulaire (2) est dimensionnée de telle sorte que dans l'état implanté de la prothèse valvulaire, les éléments d'ancrage (4a, 4b, 4c, 4d) sont réalisés pour coincer entre eux et la paroi extérieure du corps annulaire (2) en particulier du tissu des feuillets valvulaires et de cordages tendineux, et comportant
- des éléments d'ancrage (6a, 6b, 6c, 6d) côté oreillette qui s'étendent à l'extérieur du corps annulaire (2) et sensiblement parallèlement à sa paroi extérieure (2a) et qui sont reliés au corps annulaire (2) de façon analogue auxdits éléments d'ancrage (4a, 4b, 4c, 4d) côté ventriculaire,
**caractérisée en ce que**
la longueur axiale (LV, LAT) des éléments d'ancrage (4a, 4b, 4c, 4d, 6a, 6b, 6c, 6d) est réglable.

2. Prothèse valvulaire selon la revendication 1,
dans laquelle
les éléments d'ancrage (4a, 4b, 4c, 4d) s'étendent du côté ventriculaire au moins le long de la moitié de la longueur axiale (LR) du corps annulaire (2).

3. Prothèse valvulaire selon la revendication 1 ou 2,
dans laquelle
les éléments d'ancrage (4a, 4b, 4c, 4d) sont arrondis à leurs extrémités, en particulier sous forme d'anneau ou d'anneau partiel.

4. Prothèse valvulaire selon l'une des revendications précédentes,
dans laquelle
les éléments d'ancrage (6a, 6b, 6c, 6d) reliés du côté oreillette au corps annulaire s'étendent sur moins de la moitié de la longueur axiale (LR) du corps annulaire (2).

5. Prothèse valvulaire selon l'une des revendications précédentes,
dans laquelle
les éléments d'ancrage (6a, 6b, 6c, 6d) reliés du côté oreillette au corps annulaire (2) sont arrondis à leurs extrémités, en particulier sous forme d'anneau ou d'anneau partiel.

6. Prothèse valvulaire selon l'une des revendications précédentes,
dans laquelle
le corps annulaire (2) est en forme tronconique, en particulier avec un diamètre plus grand du côté oreillette que du côté ventriculaire.

7. Prothèse valvulaire selon l'une des revendications précédentes,
dans laquelle
le corps annulaire (2) est constitué en un tressage de fils, en particulier de fils en forme de zigzag.

8. Prothèse valvulaire selon l'une des revendications précédentes,
dans laquelle
les éléments d'ancrage (4a, 4b, 4c, 4d ; 6a, 6b, 6c, 6d) sont reliés au corps annulaire chacun par deux entretoises écartées sur la périphérie du corps annulaire.
